Europäisches Patentamt

**European Patent Office**

Office européen des brevets

(19)

(11) Veröffentlichungsnummer: **0 178 315**
**B1**

(12) **EUROPÄISCHE PATENTSCHRIFT**

(45) Veröffentlichungstag der Patentschrift: 24.10.90

(21) Anmeldenummer: 85901980.4

(22) Anmeldetag: 25.04.85

(86) Internationale Anmeldenummer:
PCT/DE85/00131

(87) Internationale Veröffentlichungsnummer:
WO 85/05208 21.11.85 Gazette 85/25

(51) Int. Cl.⁵: **G 09 B 5/04, A 61 B 5/00**

(54) ANLAGE ZUR LERNEN NACH DER SUPERLEARNING-METHODE.

(30) Priorität: 28.04.84 DE 3415966

(43) Veröffentlichungstag der Anmeldung:
23.04.86 Patentblatt 86/17

(45) Bekanntmachung des Hinweises auf die
Patenterteilung:
24.10.90 Patentblatt 90/43

(84) Benannte Vertragsstaaten:
AT BE CH DE FR GB IT LI NL SE

(56) Entgegenhaltungen:
DE-A-1 817 745
DE-A-2 227 264
DE-A-2 810 237
FR-A-2 209 164
FR-A-2 497 983
US-A-4 354 841

(73) Patentinhaber: TP Therapy Products AG
Industriestrasse 11
CH-6300 Zug (CH)

(72) Erfinder: NIEMÖLLER, Gerhard
Bahnhofstrasse 5
D-2401 Ratekau (DE)

(74) Vertreter: Wilcken, Hugo, Dr. et al
Patentanwälte Dr. Hugo Wilcken Dipl.-Ing.
Thomas Wilcken Musterbahn 1
D-2400 Lübeck (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung geht aus von einer Lernvorrichtung, bestehend aus einem Tonbandgerät mit einem Mehrspurtonband, das wenigstens eine Spur für Lerngut aufweist und auf wenigstens einer weiteren Spur Hintergrundmusik enthält oder damit versehbar ist, mit einer Mikrofoneinrichtung zum Aufnehmen von Lerngut und gegebenenfalls der Musik, mit einem Wählschalter zum auswählen wenigstens einer Tonbandspur und mit einem mit dem Wählschalter elektrisch verbundenen und durch diesen auf wenigstens eine Tonbandspur schaltbaren, elektrischakustischen Wandler, um das Lerngut durch Abspielen des Tonbandes der lernenden Person wieder zuzuführen.

In der FR-A-2 209 164 ist eine Lernvorrichtung der vorstehend angeführten Art beschrieben. Diese Vorrichtung ist für Personen gedacht, die auf dem musikalischen Gebiet des Gesangs ausgebildet werden sollen. Hierfür wird ein mehrspuriges Tonband als Tonträger verwendet, das teilweise bespielt und teilweise unbespielt ist. Z.B. ist eine Spur mit dem Gesangsvortrag eines Gesangslehrers oder einer professionellen Gesangsperson und eine weitere Spur mit einer Musikbegleitung versehen. Ein so vorbereitetes Tonband wird zu Lernzwecken von einer lernenden Person auf einer dritten Spur besungen. Hierbei muß aber die lernende Person einerseits ihren Part im wesentlichen bereits können, also vorher gelernt haben, bevor sie dann die dritte oder eine dafür vorgesehene Tonspur besingen kann, und zwar möglichst in Übereinstimmung mit dem professionellen Gesang, den sie währenddessen mithört. Das Wiederabspielen des so aufgenommenen Tonbandes zeigt dann den Lernerfolg nur dahingehend, ob die lernende Person die angestrebte Vollendung des gelernten Partes erreicht hat bzw. nur dieses kann mit dieser vorbekannten Vorrichtung erreicht werden. Andererseits ist hierbei natürlich klar, daß sich der Atemrhythmus der lernenden Person nicht nach den Takten des Musikstückes richtet, sondern nach dem Text bzw. der Phrasierung des Musikstückes, das heißt Atmung- und Musiktakte stehen in keinem vorgegebenen Zusammenhang. Hinsichtlich der Entspannung bei der Lernaufnahme hat die Person keinerlei Anhaltspunkte als nur das eigene Gefühl.

In der US-A-4 354 841 ist ein vierspuriges Tonband für sprachliches Lerngut offenbart, und zwar zum Erlernen einer Fremdsprache. Auf der einen Spur sind Textstellen mit dazwischenliegenden Leerstellen in der Heimatsprache und auf einer anderen Spur sind die Textstellen mit dazwischenliegenden Leerstellen in der zu erlernenden Fremdsprache aufgenommen, während die beiden anderen Spuren mit Hintergrundmusik versehen sind. Die Textstellen der einen Spur liegen dabei den Leerstellen der anderen Spur gegenüber, wobei die Leerstellen so groß sind, daß Überschneidungen der Textstellen nicht vorkommen. Dieses mit einem herkömmlichen Wiedergabegerät abspielbare Tonband ist bereits vollständig fertig vorbereitet und kann von der lernenden Person nicht verändert werden, das heißt die Person hatte bei der Präparierung des Lernstoffes keinerlei Einfluß. Die somit festliegenden und unveränderbaren Takte der Abschnitte des sprachlichen Lerngutes und der Leerstellen sind nicht für jede Person geeignet, da jede Person ein anderes Verhältnis von Atemrhythmus und Entspannungszustand zueinander beim Lernen hat. Der Lernerfolg mit einem so vorbereiteten Tonband ist daher eingeschränkt.

Eine weitere Lernmethode ist das sogenannte Superlearning. Dies ist eine Lernmethode, mit der man seine Leistung im Vergleich zum herkömmlichen Lernen von Text, Vokabeln und dergleichen um ein Vielfaches steigern kann. Voraussetzung für eine erfolgreiche Anwendung dieser Methode ist das Einhalten einer Reihe von verschiedenen Bedingungen.

Einerseits muß der Lernende beim Lernvorgang völlig entspannt sein, während andererseits das Lerngut in einem bestimmten Takt bzw. Rhythmus auf das Tonband aufgespielt und beim Lernvorgang auch in diesem Rhythmus wieder abgespielt werden muß. Beispielsweise kann ein solcher Rhythmus eine Dauer von acht Sekunden haben, wobei jeweils während vier Sekunden das Lerngut oder Textteile hiervon vorgetragen werden und danach eine Pause von vier Sekunden ohne Lernvortrag folgt. Wichtig ist es dabei ebenfalls, daß der Lernende sich mit seiner Atemtechnik an diesen Rhythmus anpaßt.

Abgesehen davon, daß auf dem Markt in dieser Form bespielte Tonbandcassetten erhältlich sind, besteht aber auch die Möglichkeit, daß man das Lerngut selbst auf das Tonband aufsprechen kann. Um hierbei allerdings die vorher angesprochenen Bedingungen erfüllen zu können, ist es bekannt, noch ein Tonband mit dem aufgespielten Zeittakt und ein weiteres Tonband mit der sogenannten Superlearning-Musik, die lernfördernd ist, zu verwenden. Hieraus folgt, daß man bisher eine Anlage mit zumindest drei Tonbandgeräten bzw. Cassettenrecordern und außerdem ein Mischpult benötigt, wenn man die einzelnen erforderlichen Komponenten sinnvoll miteinander verbinden und anwenden will.

Die Aufgabe der Erfindung besteht in der Schaffung einer Lernvorrichtung für erhöhte Lernleistung zum Selbstaufnehmen des Lerngutes, wobei die Vorrichtung weniger aufwending sowie einfach und sicher in ihrer Handhabung und Bedienung ist.

Die Lösung dieser Aufgabe geht von einer Lernvorrichtung der einleitend angeführten Art aus und kennzeichnet sich weiter dadurch, daß mit dem Wählschalter ein Taktgenerator zur akustischen Darstellung von selbstgewählten Takten und bei Bedarf ein über eine Elektrode mit der lernenden Person verbindbares Biofeedbackgerät zur akustischen Darstellung des Entspannungszustand der lernenden Person direkt elektrisch verbunden sind, um die entsprechenden Signale einzeln oder zusammen der lernenden Person über den Wandler zuzuführen, während diese das Lerngut innerhalb der selbstgewählten Takte auf das Tonband aufspricht, und daß der Taktgenerator und das Biofeedbackgerät mit ihrer Einrichtung zur Signalgabe zusammen mit dem übrigen Tonbandgerät der Vorrichtung zu einer kompakten Einheit zusammengefaßt sind.

Eine solche Lernvorrichtung zum Selbstaufnehmen von Lerngut, die auch zur Wiedergabe des Lerngutes verwendet werden kann, kommt also mit einem einzelnen Mehrspur-Tonbandgerät und nur einem mehrspurig bespielbaren Tonband aus, wobei die Aufzeichnungen, insbesondere das Textlerngut und die lernfördernde Hintergrundmusik, auf verschiedene Spuren wie bei herkömmlichen Tonbandgeräten gemischt werden können. Das Tonbandgerät und alle sonstigen Vorrichtungskomponenten werden in einfacher Weise über den Wählschalter wahlweise einzeln oder in Kombination auf den Wandler geschaltet. Somit entfallen auch die zeitaufwendigen und umständlichen Vorbereitungshandlungen, die für die Benutzung der bisherigen Anlagen erforderlich sind. Ferner kann die lernende Person mit der kompakten und einfach aufgebauten Lernvorrichtung nach der Erfindung ihren ganz persönlichen Lernzustand schnell und sicher herbeiführen und dann jeglichen Lerntext in Übereinstimmung von Atmung, Taktrhythmus für den Lerntext und körperlicher Entspannung für sich selbst lerngerecht auf das Tonband aufsprechen. Dadurch können ihre individuellen Lernvoraussetzungen jederzeit optimal berücksichtigt werden, was zu einem weiter verbesserten Lernerfolg führt.

Außerdem ist die kompakte erfindungsgemäße Lernvorrichtung kostengünstig herstellbar und läßt sich bei Nichtgebrauch platzsparend aufbewahren.

In der anliegenden Zeichnung ist ein Ausführungsbeispiel der Erfindung schematisch und vereinfacht in Form eines Blockschaltbildes dargestellt.

Als Tonbandgerät wird beispielsweise ein vierspuriger Cassettenrecorder 1 verwendet, an den ein Mikrofon 2 angeschlossen ist, so daß auf zwei Spuren des Tonbandes das Lerngut aufgenommen werden kann. Die beiden anderen Spuren sind für lernfördernde bzw. für die Superlearning-Musik vorgesehen.

An den beiden Ausgängen a und b des Gerätes 1 wird das textliche Lerngut und von den beiden anderen Ausgängen c und d die Musik wahlweise abgenommen und über vier zu einem Kabel 3 zusammengefaßte Leitungen sowie über einen entsprechend einzustellenden Wählschalter 4 zu einem Wandler 5 geleitet werden, der zweckmäßigerweise als Kopfhörer ausgebildet ist. Anstelle eines Kopfhörers kann allerdings auch ein Lautsprecher eingesetzt werden, obwohl dieser meist nicht eine gleich gute Konzentration des Lernenden zuläßt wie ein Kopfhörer.

Mit einer Steuerung 6, die an die zu den Ausgängen bzw. Anschlüssen a und b führenden Leitungen angeschlossen ist und vorteilhaft in das Gerät integriert sein sollte, kann bei Bedarf die Lautstärke des abgespielten Lerngutes bzw. Sprachvortrages automatisch und periodisch verändert werden, um die Lernkonzentration weiter zu steigern.

Mit dem Schalter 4 können außerdem die akustischen Ausgänge e und f eines Biofeedbackgerätes 7 bzw. eines üblichen Taktgenerators 8 über die Leitungen 9 bzw. 10 und 11 auf den Kopfhörer 5 geschaltet werden, während an weiteren Ausgängen g und h der Komponenten 7 bzw. 8 Einrichtungen 12 bzw. 13 angeschlossen sind, die zur optischen Darstellung der von diesen Komponenten abgegebenen Signale dienen, wobei die akustischen und optischen Signale dieselbe Aussage beinhalten. Für den hier vorliegenden Zweck benötigte Biofeedbackgeräte sind an sich bekannt und daher hier nicht im einzelnen beschrieben. Sie arbeiten meist nach dem Prinzip, daß beispielsweise an der Hand oder am Arm der betreffenden Person der relative Hautwiderstand gemessen und angezeigt wird, wobei der Entspannungszustand umso größer ist, je geringer der Hautwiderstand ist.

Mit der beschriebenen Lernvorrichtung kann folgendermaßen gearbeitet werden.

Wenn das Tonband nicht schon werksseitig vom Hersteller auf den beiden für die Musik vorgesehenen Spuren bespielt ist, muß dies selbst gemacht werden. Dann wird das Lerngut mit dem Mikrofon 2 auf einer freien Spur des Tonbandgerätes aufgenommen, und zwar in dem Rhythmus und Takt, den der in Betrieb gesetzte Taktgenerator 8 über den Kopfhörer 5 akustisch oder über die Anzeigeeinrichtung 13 optisch vorgibt. Wie schon erwähnt wurde, kann hierbei ein Rhythmus von 8 s selbst gewählt werden, indem jeweils nach 4 s Text eine Pause von 4 s folgt. Um den hierzu erforderlichen Entspannungszustand sicherzustellen, ist bei Bedarf das Biofeedbackgerät 7, das über eine Elektrode mit der lernenden Person verbunden ist, über den Wählschalter 4 zugeschaltet, so daß die lernende Person klar entscheiden kann, ob ihr geeignet erscheinender Entspannungszustand mit den gewählten Takten übereinstimmt, die gegebenenfalls zu ändern sind. Besteht zwischen Entspannung und Takten bzw. Rhythmus volle Übereinstimmung, wird das Lerngut auf die vorgesehene Tonbandspur aufgesprochen.

Wenn das Lerngut aufgesprochen ist, wird zur Vorbereitung des eigentlichen Lernvorganges wiederum mit dem Entspannungstraining begonnen, wobei der jeweils eingetretene Entspannungszustand wieder vom Biofeedbackgerät 7 optisch über die Anzeigeeinrichtung 12 oder bei entsprechender Stellung des Schalters 4 auch akustisch über den Kopfhörer 5 dargestellt wird.

Nachdem der Lernende einen entsprechend tiefen Zustand der Entspannung erreicht hat, schließt er den Kopfhörer 5 mit dem Schalter 4 an das Tonbandgerät 1 an und schaltet dieses so ein, daß er sich zunächst das Lerngut ohne Musik anhört. Hierbei ist es wichtig, im richtigen Rhythmus zu atmen, so daß zur Kontrolle der Taktgenerator 8 eingeschaltet sein sollte, was allerdings erfordert, daß der Kontrolltakt und der Takt bzw. Rhythmus des abgespielten Lerngutes synchron eingestellt sind. Außerdem kann zur Kontrolle des bestehenden Eintspannungszustandes auch das Biofeedbackgerät 7 eingeschaltet bleiben, so daß jederzeit erkennbar ist, ob der Lernvorgang evtl. zur Durchführung weiterer Entspannungsübungen unterbrochen werden muß. Im übrigen ist es klar, daß der mehrpolige Schalter 4 so ausgelegt ist, daß mit ihm alle genannten Betriebsvariationen eingestellt werden können.

Wenn das Lerngut abgehört ist, wird es noch einmal abgespielt, wobei jetzt aber die Superlearning-Musik am Gerät 1 dem zu lernenden Text zugeschaltet und dem Lernenden zusammen mit dem Text über den Kopfhörer 5 dargeboten wird. Nach dem zweiten Abhören des Textes ist der Lernvorgang beendet. Ein solches Vorgehen optimiert den Lernvorgang, da es die Entspannung und die Konzentration des Lernenden erhält.

Abschließend sei noch darauf hingewiesen, daß die Lernvorrichtung zweckmäßigerweise zu einer kompakten Einheit zusammengefaßt ist, indem die jeweiligen Vorrichtungskomponenten in und an einem einzelnen Gehäuse untergebracht bzw. angebracht werden. Andererseits besteht auch die Möglichkeit, die jeweiligen Komponenten auf einem Träger oder einer Grundplatte zu montieren.

**Patentansprüche**

1. Lernvorrichtung, bestehend aus einem Tonbandgerät (1) mit einem Mehrspurtonband, das wenigstens eine Spur für Lerngut aufweist und auf wenigstens einer weiteren Spur Hintergrundmusik enthält oder damit versehbar ist, mit einer Mikrofoneinrichtung (2) zum Aufnehmen von Lerngut und gegebenenfalls der Musik, mit einem Wählschalter (4) zum Auswählen wenigstens einer Tonbandspur und mit einem mit dem Wählschalter elektrisch verbundenen und durch diesen auf wenigstens eine Tonspur schaltbaren, elektrisch-akustischen Wandler (5), um das Lerngut durch Abspielen des Tonbandes der lernenden Person wieder zuzuführen, dadurch gekennzeichnet, daß mit dem Wählschalter (4) ein Taktgenerator (8) zur akustischen Darstellung von selbstgewählten Takten und bei Bedarf ein über eine Elektrode mit der lernenden Person verbindbares Biofeedbackgerät (7) zur akustischen Darstellung des Entspannungszustandes der lernenden Person direkt elektrisch verbunden sind, um die entsprechenden Signale einzeln oder zusammen der lernenden Person über den Wandler (5) zuzuführen, während diese das Lerngut innerhalb der selbstgewählten Takte auf das Tonband aufspricht, und daß der Taktgenerator (8) und das Biofeedbackgerät (7) mit ihrer Einrichtung zur Signalgabe zusammen mit dem übrigen Tonbandgerät (1, 4) der Vorrichtung zu einer kompakten Baueinheit zusammengefaßt sind.

2. Vorrichtung nach Anspruch 1, dadurch gekennzeichnet, daß der Taktgenerator (8) und das Biofeedbackgerät (7) zusätzlich mit Einrichtungen (12, 13) zur optischen Darstellung des Taktes bzw. des Entspannungszustandes ausgestattet sind.

**Revendications**

1. Installation d'étude, composée d'un magnétophone (1) comportant une bande sonore à pistes multiples, qui présente au moins une piste pour la matière à étudier et qui, sur au moins une autre piste, contient une musique de fond ou peut en être pourvue, comportant un appareil formant microphone (2) pour enregistrer la matière à étudier et éventuellement la musique, ainsi qu'un commutateur sélecteur (4) pour choisir au moins une piste de bande sonore et un transducteur électroacoustique (5), relié électriquement au commutateur sélecteur et susceptible d'être commuté par celui-ci sur au moins une piste sonore pour faire entendre à nouveau, à la personne qui étudie, la matière à étudier par audition de la bande sonore, caractérisée en ce que sont directement reliés électriquement au commutateur sélecteur (4) un générateur de rythmes (8) destiné à la représentation acoustique des rythmes choisis par la personne et, si nécessaire, un dispositif de rétroaction biologique (7) (ou de "biofeedback") destiné à la représentation acoustique de l'état de détente de la personne qui étudie, afin de faire entendre les signaux, à l'aide du transducteur (5), séparément ou ensemble à la personne qui étudie pendant que celle-ci enregistre sur la bande sonore la matière à étudier à l'intérieur des rythmes choisis par elle, et en ce que le générateur de cycles (8) et le dispositif de rétroaction biologique (7) avec son dispositif d'envoi de signaux sont groupés en une unité modulaire compacte avec le magnétophone additionnel (1, 4) du dispositif.

2. Installation selon la revendication 1, caractérisé en ce que le générateur de rythmes (8) et le dispositif de rétroaction biologique (7) sont de plus équipés d'appareils (12, 13) destinés à la représentation optique du rythme ou de l'état de détente.

**Claims**

1. A learning device, comprising a tape recorder (1) having a multitrack tape which has at least one track for study material and contains, or may be provided with, background music on at least one other track, comprising a microphone system (2) for recording study material and music if applicable, having a selector switch (4) for selection of at least one tape track and having an electroacoustic transducer (5) electrically connected to the selector switch and switchable thereby to at least one tape track, to allow the study material to be rechannelled to the person studying by playback of the tape, characterised in that directly electrically connected to the selector switch (4) are a pulse generator (8) for acoustic reproduction of self-selected time periods and if need be a biofeedback device (7), connectable to the person studying via an electrode, for acoustic reproduction of the state of relaxation of the person studying, for the purpose of feeding the corresponding signals singly or together via the transducer (5) to the person studying whilst the latter records the instructional material on the tape within the self-selected time periods and that the pulse generator (8) and the biofeedback device (7) with its signal emission system together with the remaining tape recorder (1, 4) of the device are combined into a compact structural unit.

2. A device according to claim 1, characterised in that the pulse generator (8) and the biofeedback device (7) are additionally provided with devices (12, 13) for the optional display of the time periods or of the relaxation condition.